# EUROPEAN PATENT SPECIFICATION

(11) **EP 3 259 368 B1**
(45) Date of publication and mention of the grant of the patent: **25.09.2019**
(21) Application number: 16706260.3
(22) Date of filing: 04.02.2016
(51) Int. Cl.: C12Q 1/6883

(54) **METHODS FOR DIAGNOSING CHRONIC VALVULAR DISEASE**
VERFAHREN ZUR DIAGNOSTIZIERUNG EINER CHRONISCHEN HERZKLAPPENERKRANKUNG
PROCÉDÉS POUR DIAGNOSTIQUER UNE VALVULOPATHIE CHRONIQUE

(30) Priority: 17.02.2015 US 201562117189 P
(43) Date of publication of application: 27.12.2017
(73) Proprietor: Société des Produits Nestlé S.A., 1800 Vevey (CH)
(72) Inventor: LI, Qinghong, Chesterfield, Missouri 63005 (US)
(74) Representative: Elkington and Fife LLP
(86) International application number: PCT/IB2016/050583
(87) International publication number: WO 2016/132244

(56) References cited:
- MAGDALENA HULANICKA ET AL: "Plasma miRNAs as potential biomarkers of chronic degenerative valvular disease in Dachshunds", BMC VETERINARY RESEARCH, BIOMED CENTRAL, LONDON, GB, vol. 10, no. 1, 26 September 2014 (2014-09-26), page 205, XP021200270, ISSN: 1746-6148, DOI: 10.1186/S12917-014-0205-8
- CAROLA STEUDEMANN ET AL: "Detection and comparison of microRNA expression in the serum of Doberman Pinschers with dilated cardiomyopathy and healthy controls", BMC VETERINARY RESEARCH, BIOMED CENTRAL, LONDON, GB, vol. 9, no. 1, 17 January 2013 (2013-01-17), page 12, XP021142477, ISSN: 1746-6148, DOI: 10.1186/1746-6148-9-12
- LI QINGHONG ET AL: "Expression Profiling of Circulating MicroRNAs in Canine Myxomatous Mitral Valve Disease.", INTERNATIONAL JOURNAL OF MOLECULAR SCIENCES 2015, vol. 16, no. 6, 2015, pages 14098-14108, XP002756462, ISSN: 1422-0067

## Description

### BACKGROUND OF THE INVENTION

### Field of the Invention

The invention relates generally to the use of microRNA (miRNA) associated with chronic valvular disease for diagnosing and predicting chronic valvular disease in a canine.

### Description of Related Art

Cardiac disease is one of the most common disorders in dogs. Chronic Valvular Disease (CVD), also known as degenerative mitral disease (DMVD), affects approximately 9% of all dogs, increasing with age such that the overall cumulative incidence is greater than 40%. CVD is characterized by a progressive degeneration and deformation of the atrioventricular valves, most commonly the mitral valves, resulting in early mitral valve insufficiency. This in turn leads to the appearance of a systolic heart murmur due to mitral regurgitation, wherein inadequate closure of the mitral valve causes blood to flow back to the left atrium. The affected dogs finally develop left atrioventricular volume overload, pulmonary edema, atrial dilatation and supraventricular arrhythmias.

Although surgical or medical treatment of affected valves is possible, nutritional intervention is preferred by pet owners. Early detection and treatment are imperative however detection can be difficult due to the lack of symptoms. A patent application (PCT/US201344011) (published as WO 2014/196957) describes methods for diagnosing CVD using differentially expressed genes and metabolites. However, gene expression methods require collection of cardiac tissues which is considered invasive. In addition, mRNA is not very stable and can be difficult to handle experimentally. Further, metabolite measurement from blood serum can be difficult depending on the biochemical nature and stability of the compounds. Hulanicka et al. (BMC Veterinary Research 2014, 10:205) describe plasma miRNAs as potential biomarkers of chronic degenerative valvular disease in Dachshunds.

Therefore there remains a need for diagnosing and predicting CVD in animals to provide the most appropriate and effective level of treatment.

### SUMMARY OF THE INVENTION

It is, therefore, an object of the present invention to provide an *in vitro* use of miRNA for diagnosing and predicting chronic valvular disease in canines.

This and other objects are achieved using microRNA (miRNA) comprising of miR-103, miR-98, let-7c, let-7b, miR-302d, miR-380, miR-874, miR-582, miR-490, miR-329b, miR-487b, and combinations thereof for diagnosing chronic valvular disease in a canine. The use preferably comprises the steps of analyzing a biological sample obtained from a canine for an amount of an miRNA associated with chronic valvular disease, wherein the miRNA is selected from the group consisting of miR-103, miR-98, let-7c, let-7b, miR-302d, miR-380, miR-874, miR-582, miR-490, miR-329b, miR-487b, and combinations thereof; comparing the amount of the miRNA identified in the sample obtained from the canine to a corresponding amount of the miRNA present in a sample from one or more comparable control animals that do not suffer from chronic valvular disease; and diagnosing the canine with chronic valvular disease if the miRNA found in the canine's sample is differentially expressed in the control animal's sample.

Other and further objects, features, and advantages of the present invention will be readily apparent to those skilled in the art.

### DETAILED DESCRIPTION OF THE INVENTION

### Definitions

The term "animal" means any animal susceptible to or suffering from chronic valvular disease, including human, avian, bovine, canine, equine, feline, hircine, lupine, murine, ovine, or porcine animals.

The terms "miRNA" or "biomarker" mean a small single-stranded non-coding RNA molecule, including those containing about 21-25 nucleotides, the levels or intensities of which are measured in a biological sample, that may be used as markers to diagnose a disease state.

The term "differential expression" or "differentially expressed" means increased or upregulated miRNA expression or means decreased or downregulated miRNA expression as detected by the absence, presence, or change in the amount of miRNA in a sample.

The term "comparable control animal" means an animal of the same species and type or an individual animal evaluated at two different times.

The term "corresponding amount" means an amount of an miRNA from a comparable control animal that corresponds to the miRNA for an animal being diagnosed with chronic valvular disease, where the miRNA is associated with chronic valvular disease.

The term "companion animals" means domesticated animals such as dogs, cats, birds, rabbits, guinea pigs, ferrets, hamsters, mice, gerbils, pleasure horses, cows, goats, sheep, donkeys, pigs, and more exotic species kept by humans for company, amusement, psychological support, extrovert display, and all of the other functions that humans need to share with animals of other species. In one aspect, companion animal can refer to a dog or cat. In one specific aspect, a companion animal can refer to a dog.

The term "diagnosing" means determining if an animal is suffering from or predicting if the animal is susceptible to developing chronic valvular disease.

The term "early stage" means stage B heart failure (HF) with mild to moderate cardiac enlargement but no clinical signs of heart failure (HF) based upon the guidelines for diagnosis of HF from American College of Veterinary Internal Medicine (ACVIM).

The term "end stage" means stage C HF with clinical signs of moderate HF based upon the ACVIM guidelines.

As used herein, ranges are used herein in shorthand, so as to avoid having to list and describe each and every value within the range. Any appropriate value within the range can be selected, where appropriate, as the upper value, lower value, or the terminus of the range.

As used herein, the singular form of a word includes the plural, and vice versa, unless the context clearly dictates otherwise. Thus, the references "a", "an", and "the" are generally inclusive of the plurals of the respective terms. For example, reference to "a method" includes a plurality of such "methods." Similarly, the words "comprise", "comprises", and "comprising" are to be interpreted inclusively rather than exclusively. Likewise the terms "include", "including" and "or" should all be construed to be inclusive, unless such a construction is clearly prohibited from the context.

The methods and compositions and other advances disclosed here are not limited to particular methodology, protocols, and reagents described herein because, as the skilled artisan will appreciate, they may vary. Further, the terminology used herein is for the purpose of describing particular embodiments only, and is not intended to, and does not, limit the scope of that which is disclosed or claimed.

Unless defined otherwise, all technical and scientific terms, terms of art, and acronyms used herein have the meanings commonly understood by one of ordinary skill in the art in the field(s) of the invention, or in the field(s) where the term is used.

The discussion of patents, patent applications, publications, technical and/or scholarly articles, and other references cited or referred to herein is intended merely to summarize the assertions made therein. No admission is made that any such patents, patent applications, publications or references, or any portion thereof, are relevant, material, or prior art. The right to challenge the accuracy and pertinence of any assertion of such patents, patent applications, publications, and other references as relevant, material, or prior art is specifically reserved.

### The Invention

The present inventors have discovered that miRNA described herein can be present in the biological sample of a canine and that the amount of the miRNA in the sample can serve as a biochemical indicator for diagnosing chronic valvular disease by indicating or predicting the threshold for chronic valvular disease. As such, the present discovery allows veterinary and other clinicians to perform tests for these "biomarkers" in a sample and determine whether the animal is susceptible to or suffering from chronic valvular disease and whether there is a need for further diagnostics or treatment. Having established the need for further diagnostics or treatments, the cost and risk of such further diagnostics or treatments can be justified.

In accordance with the above, the invention provides an *in vitro* use of microRNA (miRNA) comprising of miR-103, miR-98, let-7c, let-7b, miR-302d, miR-380, miR-874, miR-582, miR-490, miR-329b, miR-487b, and combinations thereof for diagnosing chronic valvular disease in a canine. In one embodiment, the use may comprise analyzing a biological sample obtained from the canine for an amount of a microRNA (miRNA) associated with chronic valvular disease, comparing the amount of the miRNA identified in the sample obtained from the canine to a corresponding amount of the miRNA present in a sample from one or more comparable control animals that do not suffer from chronic valvular disease, and diagnosing the canine with chronic valvular disease if the amount of the miRNA found in thecanine's sample is differentially expressed in the control animal's sample, wherein the miRNA is selected from the group consisting of miR-103, miR-98, let-7c, let-7b, miR-302d, miR-380, miR-874, miR-582, miR-490, miR-329b, miR-487b, and combinations thereof.

In one embodiment, the use for diagnosing chronic valvular disease in a canine can comprise analyzing a biological sample obtained from the canine for an amount of an miRNA associated with chronic valvular disease, comparing the amount of the miRNA identified in the sample obtained from the canine to a corresponding amount of the miRNA present in a sample from one or more comparable control animals that do not suffer from chronic valvular disease, and diagnosing the canine with chronic valvular disease if the amount of the miRNA found in the canine's sample is greater than the amount of the miRNA present in the control animal's sample, wherein the miRNA is selected from the group consisting of miR-103, miR-98, let-7c, let-7b, and combinations thereof.

In another embodiment, the use for diagnosing chronic valvular disease in a canine can comprise analyzing a biological sample obtained from the canine for an amount of an miRNA associated with chronic valvular disease, comparing the amount of the miRNA identified in the sample obtained from the canine to a corresponding amount of the miRNA present in a sample from one or more comparable control animals that do not suffer from chronic valvular disease, and diagnosing the canine with chronic valvular disease if the amount of the miRNA found in the canine's sample is less than the amount of the miRNA present in the control animal's sample, wherein the miRNA is selected from the group consisting of miR-302d, miR-380, miR-874, miR-582, miR-490, miR-329b, miR-487b, and combinations thereof.

In various embodiments, one or more comparable control animals that are not the animal being evaluated for chronic valvular disease and that have been determined not to suffer from chronic valvular disease can be evaluated for the miRNA and the results of such evaluations are used as a baseline value for comparison with the results from an animal being evaluated for the miRNA. In some embodiments, the baseline value for the miRNA can be determined by evaluating numerous comparable control animals.

In another embodiment, the amount of miRNA can be determined for an animal at various times throughout the animal's life and the results can be used to determine if the animal is susceptible to or suffering from chronic valvular disease, *e.g*., if the amount of the miRNA increases or decreases as the animal ages, the animal can be diagnosed as susceptible to or suffering from chronic valvular disease. In some embodiments, the animal can be evaluated periodically and the results for the miRNA can be recorded. Then, if a subsequent evaluation shows that the amount of the miRNA has increased or decreased since the last evaluation(s), the animal can be diagnosed as susceptible to or suffering from chronic valvular disease. In some aspects, specific changes in the miRNAs can be correlated to an early stage or end stage of chronic valvular disease.

Any sample that is of biological origin may be useful in the present invention. Examples include, but are not limited to, blood (serum/plasma), cerebral spinal fluid (CSF), urine, stool, breath, saliva, or biopsy of any tissue. In one embodiment, the sample can be a serum sample. While the term "serum" is used herein, those skilled in the art will recognize that plasma or whole blood or a sub-fraction of whole blood may also be used.

Decreased or increased expression can be measured at the miRNA level using any of the methods well known in the art for the quantitation of polynucleotides, such as, for example, PCR (including, without limitation, RT-PCR and qPCR), sequencing, Northern blotting, microarray, or other hybridization methods.

While the use of one miRNA is sufficient for diagnosing chronic valvular disease, the use of one or more, two or more, three or more, or four or more of such miRNA is encompassed within the invention. The miRNA can be evaluated and used for a diagnosis in any combination. As such, the present invention can include diagnosing CVD based on upregulated miRNA and a downregulated miRNA.

In one embodiment, the diagnosis can be based upon determining if the amount of the miRNA found in the animal's sample is greater compared to the amount of the miRNA present in the control animal's sample. In one aspect, the miRNA can include miR-103, miR-98, let-7c, or let-7b.

In one embodiment, the diagnosis can be based upon determining if the amount of the miRNA found in the animal's sample is less than compared to the amount of the miRNA present in the control animal's sample. In one aspect, the miRNA can include miR-302d, miR-380, miR-874, miR-582, miR-490, miR-329b, or miR-487b.

The differentially expressed miRNA can be statistically significant as exemplified herein. In some aspects, the statistical significance can include a p < 0.05, p < 0.01, or even p < 0.001.

In accordance with the invention, the animal is a canine, such as a dog.

### EXAMPLES

The invention can be further illustrated by the following examples, although it will be understood that these examples are included merely for purposes of illustration and are not intended to limit the scope of the invention unless otherwise specifically indicated.

### Example 1

miRNAs were identified through a differential expression profiling study comparing diseased and normal serum samples. Dogs were classified as having either a healthy heart or CVD by echocardiography performed or evaluated by a board-certified veterinary cardiologist, pathological examination of the heart or both. Dogs were classified into one of the three groups based upon their stage of heart failure (HF) using the American College of Veterinary Internal Medicine (ACVIM)/European College of Veterinary Internal Medicine (ECVIM) staging scheme: group A (stage A, at risk but unaffected, 6 dogs), group B (stage B, with mild to moderate cardiac enlargement but no clinical sign of heart failure, 6 dogs), or group C (stage C, with clinical sign of moderate heart failure, 6 dogs) (Table 1). These three groups of dogs were matched by age, body size, and sex. Blood serum was collected from each group of dogs, and was stored in -80°C until use.

**Table 1 Serum samples used in miRNA study**

| Group | Number | Disease stage classification |
|---|---|---|
| A | 6 | ACVIM stage A. At risk but unaffected |
| B | 6 | ACVIM stage B. Mild to moderate cardiac enlargement, but no clinical sign of HF |
| C | 6 | ACVIM stage C. Clinical sign of moderate HF. |

Samples were submitted to Qiagen for miRNA extraction and PCR quantification using Qiagen's miScript® miRNA PCR array system. A total of 277 known canine miRNAs were assayed. Data analysis was performed using ANOVA (Analysis of Variance). The ANOVA P values were adjusted for multiple testing and the false discovery rate (FDR) was calculated using the Benjamini and Hochberg method. Expression fold change between groups B vs. A (FC_BvA), and between groups C vs. A (FC_CvA) were calculated. Differentially expressed miRNAs were selected using the threshold of FDR<0.05. A total of 11 miRNAs were selected (Table 2).

**Table 2 Differentially expressed miRNAs**

| miRNA | P^{a} | FDR^{b} | FC^{c}_BvA | FC^{c}_CvA |
|---|---|---|---|---|
| miR-302d | 0.0010 | 0.0378 | -2.7 | -3.51 |
| miR-380 | 0.0001 | 0.0119 | -2.48 | -4.76 |
| miR-874 | 0.0016 | 0.0434 | -2.37 | -3.69 |
| miR-582 | 0.0000 | 0.0004 | -2.28 | -4.71 |
| miR-490 | 0.0005 | 0.0261 | -2.24 | -4.03 |
| miR-329b | 0.0019 | 0.0490 | -1.96 | -2.36 |
| miR-487b | 0.0012 | 0.0427 | -1.45 | -3.08 |
| miR-103 | 0.0002 | 0.0140 | 1.29 | 3.81 |
| miR-98 | 0.0014 | 0.0428 | 2.15 | 4.07 |
| let-7c | 0.0003 | 0.0218 | 2.28 | 4.67 |
| let-7b | 0.0006 | 0.0269 | 2.38 | 4.03 |

| | | | | |
|---|---|---|---|---|
| ^{a}ANOVA nominal P values; ^{b}false discovery rate; ^{c}fold changes positive numbers indicate upregulation from the control samples; negative numbers indicate downregulation from the control samples. | | | | |

In the specification, there have been disclosed typical preferred embodiments of the invention. Although specific terms are employed, they are used in a generic and descriptive sense only and not for purposes of limitation. The scope of the invention is set forth in the claims. Obviously many modifications and variations of the invention are possible in light of the above teachings. It is therefore to be understood that within the scope of the appended claims, the invention may be practiced otherwise than as specifically described.

## Claims

1. An *in* vitro use of microRNA (miRNA) selected from the group comprising of miR-103, miR-98, let-7c, let-7b, miR-302d, miR-380, miR-874, miR-582, miR-490, miR-329b, miR-487b, and combinations thereof for diagnosing chronic valvular disease in a canine.

2. The use of claim 1, wherein diagnosing chronic valvular disease in a canine comprises the steps of:
a. analyzing a biological sample obtained from the canine for an amount of a microRNA (miRNA) associated with chronic valvular disease;
b. comparing the amount of the miRNA identified in the sample obtained from the canine to a corresponding amount of the miRNA present in a sample from one or more comparable control animals that do not suffer from chronic valvular disease; and
c. diagnosing the canine with chronic valvular disease if the amount of the miRNA found in the canine's sample is greater than the amount of the miRNA present in the control animal's sample;
wherein the miRNA is selected from the group consisting of miR-103, miR-98, let-7c, let-7b, and combinations thereof.

3. The use of claim 1, wherein diagnosing chronic valvular disease in a canine comprises the steps of:
a. analyzing a biological sample obtained from the canine for an amount of a microRNA (miRNA) associated with chronic valvular disease;
b. comparing the amount of the miRNA identified in the sample obtained from the canine to a corresponding amount of the miRNA present in a sample from one or more comparable control animals that do not suffer from chronic valvular disease; and
c. diagnosing the canine with chronic valvular disease if the amount of the miRNA found in the canine's sample is less than the amount of the miRNA present in the control animal's sample;
wherein the miRNA is selected from the group consisting of miR-302d, miR-380, miR-874, miR-582, miR-490, miR-329b, miR-487b, and combinations thereof.

4. The use of claims 2 or 3, wherein the sample is a serum sample.

5. The use of claim 2 or 3, wherein the diagnosis is based upon determining the amount of two or more miRNAs associated with chronic valvular disease.

6. The use of claim 2 or 3, wherein the diagnosis is based upon determining the amount of three or more miRNAs associated with chronic valvular disease.

## Patentansprüche

1. In-vitro-Verwendung von MikroRNA (miRNA), ausgewählt aus der Gruppe, umfassend miR-103, miR-98, let-7c, let-7b, miR-302d, miR-380, miR-874, miR-582, miR-490, miR-329b, miR-487b und Kombinationen davon, zur Diagnose von chronischer Klappenkrankheit bei einem Hund.

2. Verwendung nach Anspruch 1, wobei die Diagnose von chronischer Klappenkrankheit bei einem Hund die folgenden Schritte umfasst:
a. Analysieren einer von dem Hund erhaltenen biologischen Probe auf eine Menge einer Mikro-RNA (miRNA) im Zusammenhang mit chronischer Klappenkrankheit;
b. Vergleichen der Menge der miRNA, die in der von dem Hund erhaltenen Probe identifiziert wird, mit einer entsprechenden Menge der miRNA, die in einer Probe von einem oder mehreren vergleichbaren Kontrolltieren vorhanden ist, die nicht an chronischer Klappenkrankheit leiden; und
c. Diagnostizieren chronischer Klappenkrankheit bei dem Hund, wenn die Menge der in der Probe des Hundes gefundenen miRNA größer als die Menge der in der Probe des Kontrolltieres vorhandenen miRNA ist;
wobei die miRNA ausgewählt ist aus der Gruppe, bestehend aus miR-103, miR-98, let-7c, let-7b und Kombinationen davon.

3. Verwendung nach Anspruch 1, wobei die Diagnose von chronischer Klappenkrankheit bei einem Hund die folgenden Schritte umfasst:
a. Analysieren einer von dem Hund erhaltenen biologischen Probe auf eine Menge einer Mikro-RNA (miRNA) im Zusammenhang mit chronischer Klappenkrankheit;
b. Vergleichen der Menge der miRNA, die in der von dem Hund erhaltenen Probe identifiziert wird, mit einer entsprechenden Menge der miRNA, die in einer Probe von einem oder mehreren vergleichbaren Kontrolltieren vorhanden ist, die nicht an chronischer Klappenkrankheit leiden; und
c. Diagnostizieren chronischer Klappenkrankheit bei dem Hund, wenn die Menge der in der Probe des Hundes gefundenen miRNA kleiner als die Menge der in der Probe des Kontrolltieres vorhandenen miRNA ist;
wobei die miRNA ausgewählt ist aus der Gruppe, bestehend aus miR-302d, miR-380, miR-874, miR-582, miR-490, miR-329b, miR-487b und Kombinationen davon.

4. Verwendung nach den Ansprüchen 2 oder 3, wobei die Probe eine Serumprobe ist.

5. Verwendung nach Anspruch 2 oder 3, wobei die Diagnose auf dem Bestimmen der Menge von zwei oder mehr miRNA im Zusammenhang mit chronischer Klappenkrankheit beruht.

6. Verwendung nach Anspruch 2 oder 3, wobei die Diagnose auf dem Bestimmen der Menge von drei oder mehr miRNA im Zusammenhang mit chronischer Klappenkrankheit beruht.

## Revendications

1. Utilisation *in* vitro de microARN (miARN) choisi dans le groupe comprenant miR-103, miR-98, let-7c, let-7b, miR-302d, miR-380, miR-874, miR-582, miR- 490, miR-329b, miR-487b, et leurs combinaisons pour diagnostiquer une maladie valvulaire chronique chez un canin.

2. Utilisation selon la revendication 1, dans laquelle le diagnostic d'une maladie valvulaire chronique chez un canin comprend les étapes consistant à :
a. analyser un échantillon biologique obtenu à partir du canin pour une quantité d'un microARN (miARN) associée à une maladie valvulaire chronique ;
b. comparer la quantité de miARN identifiée dans l'échantillon obtenu à partir du canin à une quantité correspondante du miARN présente dans un échantillon provenant d'un ou plusieurs animaux témoins comparables qui ne souffrent pas de maladie valvulaire chronique ; et
c. diagnostiquer le canin avec une maladie valvulaire chronique si la quantité du miARN trouvée dans l'échantillon du canin est supérieure à la quantité du miARN présente dans l'échantillon de l'animal témoin ;
dans laquelle le miARN est choisi dans le groupe constitué par miR-103, miR-98, let-7c, let-7b, et leurs combinaisons.

3. Utilisation selon la revendication 1, dans laquelle le diagnostic d'une maladie valvulaire chronique chez un canin comprend les étapes consistant à :
a. analyser un échantillon biologique obtenu à partir du canin pour une quantité d'un microARN (miARN) associée à une maladie valvulaire chronique ;
b. comparer la quantité de miARN identifiée dans l'échantillon obtenu à partir du canin à une quantité correspondante du miARN présente dans un échantillon provenant d'un ou plusieurs animaux témoins comparables qui ne souffrent pas de maladie valvulaire chronique ; et
c. diagnostiquer le canin avec une maladie valvulaire chronique si la quantité du miARN trouvée dans l'échantillon du canin est inférieure à la quantité du miARN présente dans l'échantillon de l'animal témoin ;
dans laquelle le miARN est choisi dans le groupe constitué de miR-302d, miR-380, miR-874, miR-582, miR-490, miR-329b, miR-487b, et de leurs combinaisons.

4. Utilisation selon la revendication 2 ou 3, dans laquelle l'échantillon est un échantillon de sérum.

5. Utilisation selon la revendication 2 ou 3, dans laquelle le diagnostic est fondé sur la détermination de la quantité de deux ou plus miARN associée à une maladie valvulaire chronique.

6. Utilisation selon la revendication 2 ou 3, dans laquelle le diagnostic est fondé sur la détermination de la quantité de trois ou plus miARN associée à une maladie valvulaire chronique.
